# FASCICULE DE BREVET EUROPEEN

(11) **EP 3 969 414 B1**
(45) Date de publication et mention de la délivrance du brevet: **26.03.2025**
(21) Numéro de dépôt: 20742308.8
(22) Date de dépôt: 12.05.2020
(51) Int. Cl.: C01B 21/16, C07C 45/42

(54) **PROCEDE AMELIORE DE PREPARATION D'HYDRATE D'HYDRAZINE AVEC RECYCLAGE PYRAZOLINE**
VERBESSERTES VERFAHREN ZUR HERSTELLUNG VON HYDRAZINHYDRAT MIT PYRAZOLIN-RECYCLING
IMPROVED PROCESS FOR PREPARING HYDRAZINE HYDRATE WITH PYRAZOLINE RECYCLING

(30) Priorité: 16.05.2019 FR 1905110
(43) Date de publication de la demande: 23.03.2022
(73) Titulaire: ARKEMA FRANCE, 92700 Colombes (FR)
(72) Inventeur: SAGE, Jean-Marc, 69491 PIERRE-BENITE CEDEX (FR)
(74) Mandataire: Arkema Patent
(86) Numéro de dépôt international: PCT/FR2020/050789
(87) Numéro de publication internationale: WO 2020/229774

(56) Documents cités:
- EP-A1- 0 153 216
- EP-A1- 0 179 699
- DE-T2- 3 873 185
- US-B1- 6 517 798

## Description

La présente invention concerne un procédé de préparation d'hydrate d'hydrazine. La présente invention concerne plus précisément un procédé amélioré de préparation d'hydrate d'hydrazine à partir de l'azine de la méthyl éthyl cétone obtenue en présence de méthyl éthyl cétone par oxydation de l'ammoniac par l'eau oxygénée et d'un activateur.

L'hydrazine est utilisée dans diverses applications, principalement dans la désoxygénation des eaux de chaudières (par exemple des centrales nucléaires) et sert à la préparation de dérivés pharmaceutiques et agrochimiques.

Il existe donc un besoin industriel pour la préparation d'hydrate d'hydrazine.

La production industrielle d'hydrate d'hydrazine se fait selon les procédés RASCHIG, BAYER ou à l'eau oxygénée.

Dans le procédé RASCHIG, on oxyde l'ammoniac par un hypochlorite pour obtenir une solution diluée d'hydrate d'hydrazine qu'il faut ensuite concentrer par distillation. Ce procédé peu sélectif, peu productif et très polluant, n'est presque plus utilisé.

Le procédé BAYER est une variante du procédé RASCHIG qui consiste à déplacer un équilibre chimique en piégeant, à l'aide d'acétone, l'hydrazine formée sous forme d'azine de formule suivante : (CH₃)₂C = N-N = C-(CH₃)₂.

L'azine est ensuite isolée puis hydrolysée en hydrate d'hydrazine. Les rendements sont améliorés, mais il n'y a pas d'amélioration quant aux rejets dans l'environnement.

Le procédé à l'eau oxygénée consiste à oxyder un mélange d'ammoniac et d'une cétone par l'eau oxygénée en présence d'un moyen d'activation de l'eau oxygénée pour faire directement l'azine qu'il suffit ensuite d'hydrolyser en hydrate d'hydrazine. Les rendements sont élevés et le procédé n'est pas polluant. Ce procédé à l'eau oxygénée est décrit dans de nombreux brevets, par exemple US 3 972 878, US 3 972 876, US 3 948 902 et US 4 093 656.

L'hydrolyse d'une azine en hydrate d'hydrazine est décrite dans les brevets US 4 724 133 SCHIRMANN et al., US 4 725 421 SCHIRMANN et al. et GB 1 164 460. Cette hydrolyse s'effectue dans une colonne de distillation que l'on alimente avec de l'eau et de l'azine. En tête on récupère la cétone et en pied une solution concentrée d'hydrate d'hydrazine.

Ces procédés sont aussi décrits dans ULLMANN'S ENCYCLOPEDIA OF INDUSTRIAL CHEMISTRY (1989), vol A 13, pages 182-183 et les références incluses.

Dans les procédés à l'eau oxygénée, on oxyde l'ammoniac par le peroxyde d'hydrogène en présence d'une cétone et d'un moyen d'activation du peroxyde d'hydrogène selon la réaction globale suivante, en faisant une azine :

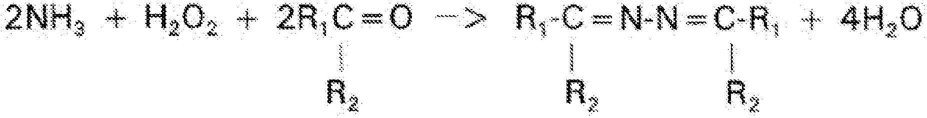

Le moyen d'activation ou activateur peut être un nitrile, un amide, un acide carboxylique ou encore un dérivé du sélénium, de l'antimoine ou de l'arsenic. Puis l'azine est hydrolysée en hydrazine et la cétone est régénérée selon la réaction suivante :

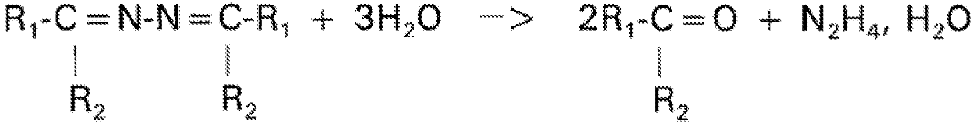

Cette hydrolyse est effectuée en réalité en deux temps, avec formation d'une hydrazone intermédiaire :

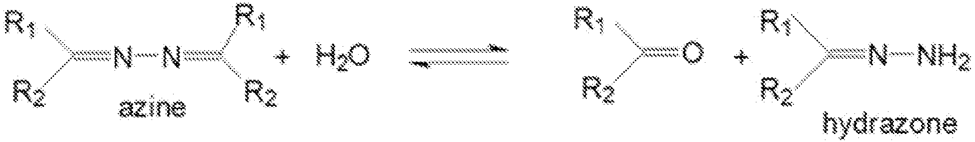

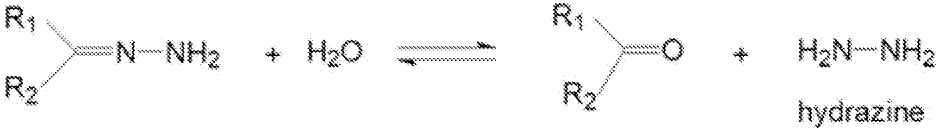

Que l'azine soit produite par un procédé à l'eau oxygénée ou un autre procédé, on utilise avantageusement la méthyl éthyl cétone parce qu'elle est peu soluble dans un milieu aqueux.

En effet, dans le procédé à l'eau oxygénée, l'azine de la méthyl éthyl cétone est relativement insoluble dans le milieu réactionnel qui est forcément aqueux puisque l'on utilise des solutions aqueuses commerciales de peroxyde d'hydrogène titrant entre 30 et 70 % en poids. Cette azine est donc facilement récupérable et séparable par simple décantation. Elle est très stable surtout en milieu alcalin, c'est-à-dire dans le milieu réactionnel ammoniacal. Dans les procédés actuels, cette azine est ensuite purifiée, puis hydrolysée dans une colonne de distillation réactive pour libérer *in fine* de la méthyl éthyl cétone en tête à recycler, et surtout une solution aqueuse d'hydrate d'hydrazine en pied, qui doit contenir aussi peu de produits carbonés que possible comme impuretés et doit être incolore.

Toutefois, dans ces procédés à l'eau oxygénée et utilisant la méthyl éthyl cétone (également appelée MEK), il se forme de faibles quantités de sous-produits et notamment des hétérocycles de la famille de la pyrazoline. En particulier, ces sous-produits sont la 3,4,5-triméthyl-5-éthyl-pyrazoline et la 3,5-diéthyl-5-méthyl-pyrazoline, de formules suivantes :

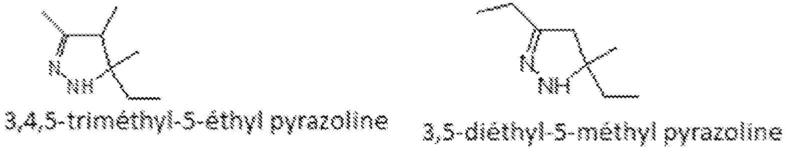

Ces composés sont des isomères de l'azine de la méthyl éthyl cétone. Ils se forment principalement dans la colonne d'hydrolyse, et sont responsables des colorations jaune, voire brune ou rouge de l'hydrazine. On peut ainsi obtenir en pied de colonne une solution d'hydrate d'hydrazine non pas incolore, mais colorée et parfois fortement, en fonction de la teneur en hétérocycles de la famille de la pyrazoline obtenue.

Ces sous-produits peuvent s'accumuler sur les plateaux de la colonne d'hydrolyse, engendrant les problèmes de coloration décrits ci-dessus et des problèmes de conduite au-delà d'une certaine concentration. De préférence, la teneur en hétérocycles de la famille de la pyrazoline est inférieure ou égale à 5 % en poids, plus préférentiellement comprise entre 1% et 3% en poids, par rapport au poids total de la phase liquide présente sur les plateaux de la colonne ou dans les parties de la colonne à hydrolyse où ladite teneur est à son maximum. Ces valeurs sont suffisantes pour éviter de colorer l'hydrate d'hydrazine produit.

Il existe donc un besoin pour un procédé industriel de préparation d'hydrate d'hydrazine amélioré quant au traitement des sous-produits tels que les hétérocycles de la famille de la pyrazoline, et en particulier la 3,4,5-triméthyl-5-éthyl-pyrazoline et la 3,5-diéthyl-5-méthyl-pyrazoline.

Le document WO 99/58445 décrit la purge discontinue des hétérocycles de la famille de la pyrazoline lors de l'étape d'hydrolyse de l'azine en hydrate d'hydrazine. Il est mentionné que ces composés se forment dans la colonne d'hydrolyse.

Toutefois, ce document ne donne aucune indication quant à un éventuel traitement de cette purge.

Il existe donc un besoin pour un procédé amélioré de préparation d'hydrate d'hydrazine dans lequel les sous-produits sont traités de façon plus économique et plus respectueuse de l'environnement.

De façon surprenante, il a été découvert que lorsque l'on recycle la purge en hétérocycles de la famille de la pyrazoline en amont de l'étape d'hydrolyse, ces sous-produits reviennent solubilisés dans l'azine à l'entrée de l'étape de distillation (e), et sont éliminés lors de cette étape de distillation. L'étape de distillation permet de façon surprenante d'éliminer les hétérocycles de la famille de la pyrazoline. De plus, les précurseurs d'hydrazine (azine et hydrazone) pouvant être contenus dans cette purge sont récupérés dans le flux d'azine et reviennent dans la colonne d'hydrolyse.

Le recyclage selon l'invention permet d'atteindre une stabilisation de la concentration en hétérocycles de la famille de la pyrazoline dans la colonne d'hydrolyse, à une concentration suffisamment basse pour obtenir une solution d'hydrate d'hydrazine incolore en pied de la colonne d'hydrolyse et ce de façon stable lorsque l'hydrolyse est opérée en continu.

Le recyclage de la purge des hétérocycles de la famille de la pyrazoline selon l'invention présente ainsi de nombreux avantages. On évite de rejeter un effluent contenant de l'azine et de l'hydrazone qui représentent des produits intermédiaires conduisant à l'hydrazine, tout en éliminant les sous-produits du procédé, sans ajouter d'opérations ou surcoût supplémentaire au procédé.

Si l'effluent contenant lesdits hétérocycles était rejeté, il serait nécessaire mettre en œuvre un traitement supplémentaire, comme par exemple une incinération ou un traitement d'oxydation avant son rejet dans l'environnement.

Le procédé tel que selon l'invention permet également de ne pas perdre les composés hydrazone et azine qui peuvent être contenus dans la purge car l'hydrazone et l'azine conduisent par hydrolyse à l'hydrate d'hydrazine.

Ainsi, la présente invention concerne un procédé de préparation d'hydrate d'hydrazine comprenant les étapes suivantes :
(a) on fait réagir l'ammoniac, le peroxyde d'hydrogène et la méthyl éthyl cétone en présence d'une solution comprenant au moins un activateur pour former une azine;
(b) on sépare à partir du mélange réactionnel issu de l'étape (a) :
   - la phase aqueuse comprenant le(s) activateur(s) ; et
   - la phase organique comprenant l'azine formée et éventuellement la méthyl éthyl cétone n'ayant pas réagi ;
(c) éventuellement on recycle à l'étape (a) la phase aqueuse après un traitement éventuel;
(d) on lave, de préférence à contre-courant, la phase organique ;
(e) on distille la phase organique lavée de façon à récupérer l'azine ;
(f) on hydrolyse l'azine pour obtenir de l'hydrate d'hydrazine et régénérer la méthyl éthyl cétone, tout en effectuant une purge des hétérocycles de la famille de la pyrazoline ;
(g) éventuellement on recycle à l'étape (a) la méthyl éthyl cétone obtenue à l'étape (f) ;
(h) on recycle la purge des hétérocycles de la famille de la pyrazoline obtenue à l'étape (f) à l'une au moins des étapes (a), (b), (c), (d) ou (g).

La purge des hétérocycles de la famille de la pyrazoline comprend évidemment lesdits hétérocycles, mais peut également comprendre de l'azine, de l'hydrazone et de l'eau. Ladite purge est notamment sous forme aqueuse.

De préférence, l'ensemble des étapes du procédé sont réalisées en continu.

On entend notamment par « hétérocycles de la famille de la pyrazoline », un cycle pyrazoline substitué sur l'un au moins de ses atomes de carbone, en particulier un cycle pyrazoline de formule (I) suivante : 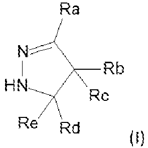 dans laquelle Ra, Rb, Rc, Rd et Re peuvent être indépendamment les uns des autres choisis parmi H, et les (C₁-C₂₀)alkyles ; l'un au moins des radicaux Ra, Rb, Rc, Rd et Re étant un (C₁-C₂₀)alkyle.

Le terme « (C₁-C₂₀)alkyle » désigne des hydrocarbures aliphatiques saturés, qui peuvent être linéaires ou ramifiés et comprennent de 1 à 20 atomes de carbone. De préférence, les alkyles comprennent de 1 à 4 atomes de carbone, voire de 1 à 2 atomes de carbone. On peut citer par exemple le méthyle et l'éthyle. Par « ramifié », on entend qu'un groupement alkyle est substitué sur la chaîne alkyle principale.

Il est notamment entendu par azine, l'azine de la méthyl éthyl cétone encore appelée mecazine.

### Etape (a) : Formation de l'azine de la méthyl éthyl cétone

Dans l'étape (a), on fait réagir l'ammoniac, le peroxyde d'hydrogène et la méthyl éthyl cétone en présence d'une solution comprenant au moins un activateur pour former une azine, c'est-à-dire l'azine de la méthyl éthyl cétone.

L'ammoniac peut être anhydre ou en solution aqueuse.

L'eau oxygénée peut être utilisée sous sa forme commerciale habituelle, par exemple en solution aqueuse entre 30 % et 90 % en poids d'H₂O₂. Avantageusement, on peut ajouter un ou plusieurs stabilisants usuels des solutions peroxydiques, par exemple de l'acide phosphorique, pyrophosphorique, citrique, nitrilo-triacétique, éthylènediaminetétracétique ou les sels d'ammonium ou de métal alcalin de ces acides. La quantité à utiliser est avantageusement comprise entre 10 et 1000 ppm et, de préférence, entre 50 et 250 ppm de l'ensemble des réactifs et de la solution comprenant au moins un activateur à l'entrée du réacteur.

Par « activateur », on entend un composé permettant d'activer l'eau oxygénée, c'est-à-dire un composé tel que l'on puisse produire de l'azine à partir d'ammoniac, d'eau oxygénée et de méthyl éthyl cétone.

Cet activateur peut être choisi parmi les oxyacides organiques ou inorganiques, leurs sels d'ammonium et leurs dérivés : anhydrides, esters, amides, nitriles, peroxydes d'acyle, ou leurs mélanges. Avantageusement, on utilise les amides, les sels d'ammonium et les nitriles.

A titre d'exemple, on peut citer :
(i} des amides d'acides carboxyliques de formule R₅COOH dans laquelle R₅ est l'hydrogène, un radical alkyle linéaire ayant de 1 à 20 atomes de carbone, ou un radical alkyle ramifié ou cyclique ayant de 3 à 12 atomes de carbone, ou un radical phényle pouvant être substitué,
(ii} des amides d'acides polycarboxyliques de formule R₆(COOH)ₙ dans laquelle R₆ représente un radical alkylène ayant de 1 à 10 atomes de carbone et n étant un nombre entier supérieur ou égal à 2, R₆ peut être une simple liaison, alors n vaut 2.

Les radicaux R₅ et R₆ peuvent être substitués par des halogènes, des groupements OH, NO₂ ou méthoxy. On peut citer aussi les amides des acides organiques de l'arsenic. Les acides organiques de l'arsenic sont, par exemple, l'acide méthylarsonic, l'acide phénylarsonic et l'acide cacodylique.

Les amides préférés sont le formamide, l'acétamide, le monochloracétamide et le propionamide, et plus préférentiellement l'acétamide.

Parmi les sels d'ammonium, on utilise avantageusement les sels d'hydracides, d'oxyacides minéraux, d'acides arylsulfoniques, d'acides de formules R₅COOH ou R₆(COOH)ₙ, R₅, R₆ et n étant définis précédemment, des acides organiques de l'arsenic. Les sels d'ammonium préférés sont le formiate, l'acétate, le monochloracétate, le propionate, le phénylarsonate et le cacodylate.

Parmi les nitriles, on peut citer avantageusement les produits de formule R₇(CN)ₙ, n pouvant varier de 1 à 5 selon la valence de R₇, R₇ est un alkyle cyclique ou non cyclique ayant de 1 à 12 atomes de carbone ou un benzyle ou un groupement pyridinyl. R₇ peut être substitué par des groupements qui ne s'oxydent pas dans le réacteur de l'étape (a), par exemple des halogènes, des groupes carboxyliques, esters carboxyliques, nitro, amine, hydroxy ou acide sulfonique.

Les nitriles préférés sont l'acétonitrile et le propionitrile.

On forme la solution comprenant au moins un activateur en mettant en solution un ou plusieurs produits choisis parmi les oxyacides organiques ou inorganiques, leurs sels d'ammonium et leurs dérivés : anhydrides, esters, amides, nitriles, peroxydes d'acyle, ou leurs mélanges tels que définis ci-dessus. Avantageusement, on utilise les amides, les sels d'ammonium ou les nitriles précédents. De façon particulièrement préférée, on utilise un seul activateur, qui est l'acétamide.

Cette solution peut être aqueuse ou à base d'un alcool ou d'un mélange d'alcool et eau. Parmi les alcools, on utilise avantageusement les alcools aliphatiques saturés ayant de 1 à 6 atomes de carbone et, de préférence, 1 à 2 atomes de carbone.

On utilise aussi avantageusement des diols et plus particulièrement des diols ayant de 2 à 5 atomes de carbone. On peut citer par exemple le glycol, le propylèneglycol, le 1,3 propanediol, le 1,3 et 1,4 butanediol et le 1,5 pentanediol.

Selon un mode de réalisation, ladite solution est une solution alcoolique d'un acide organique de l'arsenic et est décrite dans le brevet EP 0 070 155.

Selon un autre mode de réalisation, ladite solution est une solution aqueuse d'un amide d'acide faible et du sel d'ammonium correspondant à cet acide telle que décrite dans le brevet EP 0 487 160.

Ces amides d'acides faibles sont dérivés des acides carboxyliques correspondants qui ont une constante de dissociation inférieure à 3 x 10⁻³, c'est-à-dire les acides qui ont un pK supérieur à 3 en solution aqueuse à 25°C.

Pour les acides polycarboxyliques, ce sont les acides dont la constante de la première ionisation est inférieure à 3 x 10⁻³.

À titre d'exemple, on peut citer les acides carboxyliques de formule R₈COOH dans laquelle R₈ est un radical alkyle linéaire ayant de 1 à 20 atomes de carbone, ou un radical alkyle ramifié ou cyclique ayant de 3 à 12 atomes de carbone, ou un radical phényle pouvant être substitué, des acides polycarboxyliques de formule R₉(COOH)ₙ dans laquelle R₉ représente un radical alkylène ayant de 1 à 10 atomes de carbone et n étant un nombre supérieur ou égal à 2, R₉ peut être une simple liaison alors n vaut 2. Les radicaux R₈ et R₉ peuvent être substitués par des halogènes, des groupements OH, NO₂ ou méthoxy. On utilise de préférence l'acétamide, le propionamide, le n-butyramide ou l'isobutyramide.

Le sel d'ammonium correspondant de l'acétamide est l'acétate d'ammonium.

On ne sortirait pas du cadre de l'invention en formant le sel d'ammonium in situ c'est-à-dire en utilisant l'acide carboxylique correspondant qui donne par réaction avec l'ammoniac le sel d'ammonium.

Les proportions de l'amide et du sel d'ammonium correspondant peuvent varier dans de larges limites. On utilise habituellement de 1 à 25 parties du sel d'ammonium pour 5 parties d'amide et de préférence 2 à 10.

Les réactifs peuvent être utilisés en quantités stoechiométriques. Cependant, on peut utiliser par mole d'eau oxygénée 0,2 à 5 moles et de préférence 1,5 à 4 moles de méthyl éthyl cétone ; de 0,1 à 10 moles et de préférence de 1,5 à 4 moles d'ammoniac. La quantité de solution comprenant au moins un activateur peut être comprise entre 0,1 et 2 kg par mole d'eau oxygénée. Cette quantité dépend de sa qualité, c'est-à-dire de sa force catalytique ou son activité qui permet de convertir les réactifs en azine. Les proportions des réactifs fixées ci-dessus permettent d'obtenir une conversion totale de l'eau oxygénée et une production d'azine correspondant à plus de 50 % de l'eau oxygénée engagée et pouvant atteindre 90 %.

La mise en contact de l'eau oxygénée, de l'ammoniac, de la méthyl éthyl cétone avec la solution comprenant au moins un activateur peut s'effectuer de façon quelconque.

On peut opérer dans un milieu homogène ou dans un milieu qui assure au moins une solubilisation suffisante des réactifs pour pouvoir obtenir de l'azine. La réaction peut se faire dans une très large plage de température, par exemple entre 0°C et 100°C, et on opère avantageusement entre 30°C et 70°C. Bien que l'on puisse opérer à toute pression, il est plus simple d'être à la pression atmosphérique, mais on peut monter jusqu'à environ 10 bars si nécessaire pour maintenir de préférence la réaction de l'étape (a) en phase liquide.

Les réactifs peuvent être introduits simultanément ou séparément et dans un ordre quelconque dans la solution comprenant au moins un activateur.

On peut utiliser toutes sortes de réacteurs, agités ou non agités, ou même de simples capacités qu'on peut disposer en parallèle, en série, à co-courant ou à contre-courant, ou toute combinaison de ces possibilités.

On obtient ainsi, suite à la réaction de l'étape (a), un mélange réactionnel comprenant l'azine formée, éventuellement la méthyl éthyl cétone n'ayant pas réagi, éventuellement le(s) activateur(s), et éventuellement d'autres sous-produits ou impuretés.

### Etape (b) : Séparation de phases

On sépare la phase aqueuse comprenant le ou les activateur(s) de la phase organique comprenant l'azine formée et éventuellement la méthyl éthyl cétone n'ayant pas réagi par des moyens classiques tels que l'extraction liquide-liquide, la distillation, la décantation ou toute combinaison de ces possibilités. De préférence, on utilise la décantation.

La phase organique obtenue peut comprendre l'azine et de la méthyl éthyl cétone n'ayant pas réagi, de(s) l'activateur(s), et éventuellement des impuretés.

### Etape (c) : Recyclage de la phase aqueuse

Dans l'étape (c), la phase aqueuse peut être traitée avant recyclage à l'étape (a), classiquement par exemple par régénération thermique puis éventuellement concentration.

Les étapes (a), (b) et (c) sont décrites par exemple dans les brevets EP 399 866 et EP 518 728.

### Etape (d) : Lavage de la phase organique

L'étape de lavage de la phase organique obtenue à l'étape (b) est une étape pouvant être réalisée selon des techniques connues de l'homme du métier, comme indiqué par exemple dans le document WO 2018/065997 (p.13, « Organic layer processing section », second paragraphe). L'étape de lavage permet notamment de récupérer le(s) activateur(s), par exemple l'acétamide, qui serai(en)t encore présent(s) dans la phase organique.

Le lavage peut être effectué dans une colonne de lavage à contre-courant.

De préférence, le lavage est effectué à contre-courant par la purge d'hétérocycles de la famille de la pyrazoline effectuée à l'étape (f) d'hydrolyse, éventuellement après ajout d'eau. Le lavage peut être effectué par tout ou partie de ladite purge. Encore plus préférentiellement, la purge est suffisante pour effectuer le lavage, sans ajout d'eau, ce qui permet notamment d'économiser une entrée d'eau supplémentaire dans le procédé. Par exemple, la purge d'hétérocycles de la famille de la pyrazoline telle que définie ci-dessus est ajoutée en tête de colonne et la phase organique à laver est ajoutée en pied de colonne. Le(s) activateur(s) pouvant encore être contenu(s) dans la phase organique passe(nt) ainsi dans la phase aqueuse de lavage (soit la purge d'hétérocycles de la famille de la pyrazoline).

Selon un mode de réalisation, après passage dans la colonne de lavage, la phase aqueuse résultante est soit recyclée à l'étape (a) avec la phase aqueuse récupérée à l'étape (b), soit encore peut être renvoyée directement dans la colonne d'hydrolyse.

### Etape (e) : Distillation de la phase organique

L'étape de distillation de la phase organique lavée est une étape pouvant être réalisée selon des techniques connues de l'homme du métier, comme indiqué par exemple dans le document WO 2018/065997 (p.13, Organic layer processing section), notamment dans une colonne de distillation.

L'étape de distillation permet de séparer l'azine des hétérocycles de la famille de la pyrazoline tels que définis ci-dessus et d'autres impuretés lourdes, à haut point d'ébullition. On récupère ces sous-produits en pied de colonne par exemple.

L'étape de distillation sert également à séparer l'azine formée à l'étape (a) de la méthyl éthyl cétone n'ayant pas réagi, que l'on peut récupérer en tête de colonne. Il est possible de recycler la méthyl éthyl cétone ainsi récupérée à l'étape (a) de synthèse de l'azine.

Ainsi, à l'issue des étapes de lavage et de distillation, on obtient une phase organique purifiée, comprenant l'azine.

### Etape (f) : Hydrolyse de l'azine, régénération de la MEK et purge des hétérocycles de la famille de la pyrazoline

### Hydrolyse de l'azine et régénération de la méthyl éthyl cétone

L'étape d'hydrolyse s'effectue de préférence en continu, sous pression, dans une colonne à distiller réactive dans laquelle sont injectées l'eau et la phase organique comprenant l'azine provenant des étapes (d) ou (e).

L'hydrolyse peut être effectuée dans une colonne de distillation à garnissage ou à plateaux, de préférence fonctionnant sous une pression de 2 à 25 bars et avec une température de pied comprise entre 150°C et 200°C.

Bien que des colonnes classiques à garnissage puissent convenir, on utilise en général des colonnes à plateaux. Suivant le temps de séjour admis sur les plateaux et la pression, donc les températures auxquelles on opère, le nombre de plateaux peut varier énormément. Dans la pratique, lorsqu'on opère sous une pression de 8 à 10 bars, le nombre de plateaux nécessaires est de l'ordre de 40 à 50.

Suite à l'hydrolyse, on obtient :
- en tête, la méthyl éthyl cétone notamment sous forme d'un azéotrope avec l'eau, et
- en pied, une solution aqueuse d'hydrate d'hydrazine.

L'hydrolyse des azines est connue. Par exemple, E.C. GILBERT, dans un article dans le Journal of American Chemical Society vol.51, pages 3397-3409 (1929), décrit les réactions équilibrées de formation d'azine et les réactions d'hydrolyse de celle-ci et fournit les paramètres thermodynamiques du système dans le cas d'azines solubles dans l'eau. Par exemple, l'hydrolyse de l'azine de l'acétone est décrite dans US 4 724 133. S'agissant des azines insolubles dans les solutions aqueuses (par exemple l'azine de la méthyl éthyl cétone) l'hydrolyse doit être réalisée dans une colonne réactive, de telle sorte qu'en séparant continuellement la méthyl éthyl cétone en tête de colonne de distillation et l'hydrate d'hydrazine en pied de colonne, on peut parvenir à une hydrolyse totale. Bien entendu ce système est au meilleur de son fonctionnement lorsqu'on travaille en continu comme décrit dans les brevets FR 1 315 348 ou GB 1 211 54 7, ou encore le brevet US 4 725 421.

Dans tous ces brevets, la réaction est réalisée dans une colonne de distillation à garnissage ou mieux à plateaux fonctionnant sous une pression de 2 à 25 bars et avec une température de pied de 150°C à 200°C.

Lorsqu'on travaille avec de l'azine pure, c'est-à-dire obtenue par exemple à partir d'hydrate d'hydrazine et de méthyl éthyl cétone, on constate effectivement en travaillant selon ces brevets, que l'on obtient avec un bon rendement des solutions diluées d'hydrate d'hydrazine.

Dans cette colonne, s'effectuent l'hydrolyse de l'azine et la séparation de l'hydrate d'hydrazine d'avec la méthyl éthyl cétone. Ces conditions sont connues. L'homme du métier détermine facilement le nombre de plateaux ou la hauteur de garnissage, ainsi que les points d'alimentation en azine et en eau. On obtient en pied des solutions à 30 % ou même jusqu'à 45 % en poids d'hydrate d'hydrazine. Par exemple, le rapport molaire eau/azine à l'alimentation de cette colonne est au moins supérieur à la stœchiométrie et avantageusement compris entre 5 et 30, de préférence entre 10 et 20. La température du pied de colonne peut être comprise entre 150°C et 200°C, de préférence entre 175°C et 190°C. La pression est fonction de la température d'ébullition de l'azine, de l'eau et de la cétone. Une telle hydrolyse est également décrite dans US 4 725 421 et dans WO 00/37357.

### Purge des hétérocycles de la famille de la pyrazoline

On effectue selon l'invention la purge des hétérocycles de la famille de la pyrazoline lors de l'étape (f) d'hydrolyse.

Selon le nombre de plateaux ou la hauteur de garnissage, la position de l'alimentation en azine et la position de l'alimentation en eau, le reflux, la nature de l'azine etc ..., l'homme du métier peut déterminer facilement dans quelle partie de la colonne on obtient la concentration maximum en hétérocycles de la famille de la pyrazoline. Il est en effet plus simple de purger lesdits hétérocycles par un soutirage à l'endroit où leur concentration est maximum, par exemple sur un ou plusieurs plateaux. On peut faire un soutirage en continu, ou en discontinu, de préférence en continu.

La quantité à soutirer peut se déterminer facilement par analyse par chromatographie en phase gazeuse de la concentration de ces hétérocycles.

De préférence, la purge des hétérocycles de la famille de la pyrazoline est réalisée par soutirage, de préférence par un soutirage latéral en continu.

### Etape (g) : Recyclage de la méthyl éthyl cétone régénérée

On peut recycler à l'étape (a) la méthyl éthyl cétone obtenue à l'étape (f).

### Etape (h) : Recyclage de la purge des hétérocycles de la famille de la pyrazoline

De préférence l'étape (h) de recyclage est réalisée en continu. On peut recycler la purge des hétérocycles de la famille de la pyrazoline obtenue à l'étape (f), à l'une au moins des étapes (a), (b), (c), (d) ou (g) du procédé selon l'invention, de préférence à l'une au moins des étapes (c), (d) ou (g). De façon encore plus préférée, on recycle la purge des hétérocycles de la famille de la pyrazoline obtenue à l'étape (f), à l'étape (d) de lavage de la phase organique.

Ainsi, la purge des hétérocycles de la famille de la pyrazoline peut être recyclée :
- soit dans la phase aqueuse résultant de l'étape de séparation (b) qui est recyclée à l'étape (a) ;
- soit avec la méthyl éthyl cétone récupérée en tête de la colonne d'hydrolyse et qui est recyclée à l'étape (a) ;
- soit dans l'étape de lavage (d).

### Description des Figures

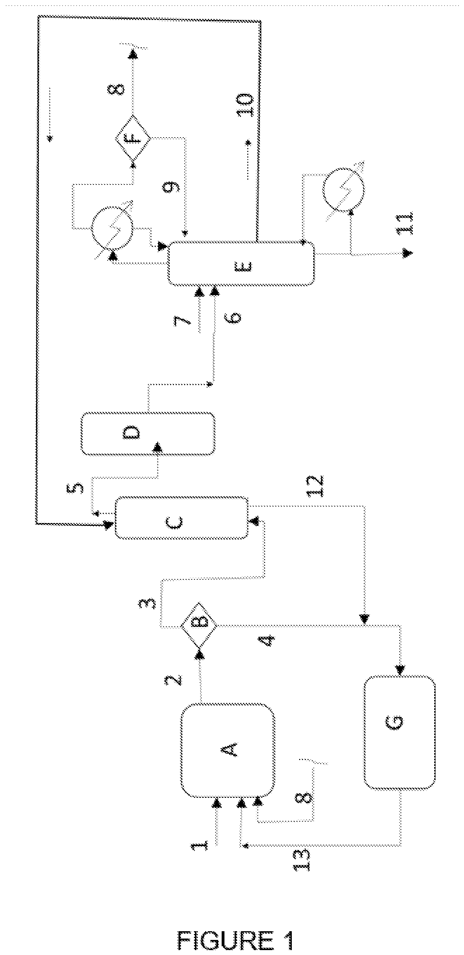

### Figure 1 : Procédé de préparation d'hydrate d'hydrazine selon l'invention

La Figure 1 représente un exemple de mise en œuvre industrielle du procédé selon l'invention.

**A** représente l'étape (a) de synthèse de la mécazine, le flux 1 comprend de l'ammoniac, de l'eau oxygénée ainsi que des appoints nécessaires en acide acétique, acétate d'ammonium ou acétamide ou encore en méthyl éthyl cétone ainsi que les divers additifs utiles lors de l'étape de synthèse, comme par exemple les stabilisants peroxydes. Le flux 13 correspond au recyclage de la phase aqueuse selon l'étape (c), après sa régénération thermique et concentration pour éliminer l'eau excédentaire. Le flux 8 correspond au recyclage de la Méthyl éthyl cétone régénérée lors de l'étape d'hydrolyse et récupérée en sortie de la colonne d'hydrolyse E selon l'étape (g).

**B** représente un décanteur en sortie de l'étape (a) de synthèse de l'azine qui reçoit le mélange réactionnel 2. Il permet de séparer la phase organique contenant la mecazine brute correspondant au flux 3 et la phase aqueuse contenant l'activateur, par exemple l'acétamide, et correspondant au flux 4 selon l'étape (b).

**C** représente une colonne de lavage à contre-courant selon l'étape (d). La phase organique, flux 3 est introduite en bas de la colonne C et est lavée à contre-courant par le flux 10 correspondant à la purge des hétérocycles de la famille de la pyrazoline extraite des plateaux de la colonne d'hydrolyse de l'azine, **E** selon l'étape (h). Le flux 12, correspondant à la phase aqueuse en sortie de la colonne de lavage C est ensuite renvoyé en section **G** correspondant à l'étape de régénération thermique et concentration de la phase aqueuse avec le flux 4.

La phase organique lavée, flux 5, est envoyée en purification sur une colonne de distillation **D** selon l'étape (e). Cette colonne permet de récupérer en tête un peu de méthyl éthyle cétone recyclée en A et permet d'éliminer en pieds les hétérocyles de la famille de la pyrazoline présents dans l'azine (non représenté).

La phase organique comprenant l'azine distillée, flux 6, est envoyée ensuite dans la colonne d'hydrolyse E. La colonne d'hydrolyse E est une colonne à distiller fonctionnant sous pression. L'azine distillée, 6, est introduite dans la colonne E ainsi que de l'eau, flux 7, nécessaire à l'hydrolyse.

Après l'étape d'hydrolyse (f), en tête, après condensation des vapeurs et décantation en F, on obtient le flux 8, comprenant principalement la méthyl éthyl cétone, de l'eau et un peu d'azine. Cette phase est recyclée à l'étape de synthèse de l'azine A.

La phase aqueuse décantée, flux 9, est renvoyée dans la colonne d'hydrolyse en tête. Le flux 11 correspond à la solution d'hydrate d'hydrazine obtenue et récupérée en pied de colonne.

Les exemples sont donnés à titre purement illustratif et ne sont pas limitatifs de l'invention.

### EXEMPLES

### Exemple 1 : Procédé de préparation selon l'invention

Le procédé tel que décrit pour la Figure 1 est mis en oeuvre.

La colonne E fonctionne dans les conditions décrites ci-après :

| | |
|---|---|
| Température en fond | 178-190°C |
| Température du condenseur en tête | 160°C |
| Pression de tête | 7,5 à 9,7 bars absolus |
| Rebouillage | Température 200°C, Pression 16 bars |
| Alimentation en azine distillée 6 | 5000 kg/heure |
| Alimentation en eau 7 | 10000 kg/heure |

Soutirage d'hydrazine en pied 12 t/h d'une solution aqueuse à 22,2% exprimé en hydrate d'hydrazine (ou 14,2% exprimé en hydrazine N₂H₄)

Après l'étape d'hydrolyse (f), en tête, après condensation des vapeurs et décantation, on soutire environ 6 500 kg/heure de phase organique, flux 8, comprenant principalement la méthyl éthyl cétone, de l'eau et un peu d'azine. Cette phase est recyclée à l'étape de synthèse de l'azine A.

La phase aqueuse décantée, flux 9, est renvoyée dans la colonne d'hydrolyse en tête.

Une purge, flux 10 est effectuée sur les plateaux de la colonne d'hydrolyse à un débit de 1477 kg/h, à l'endroit où les hétérocycles de la famille de la pyrazoline s'accumulent. Cette purge est renvoyée dans la colonne de lavage C pour laver la phase organique issue du séparateur B.

Le relevé des débits et analyses effectuées autour de la colonne de lavage C sont reportés dans le tableau 1 :

| Tableau 1 | colonne de lavage C de l'azine brute | | | | | | |
|---|---|---|---|---|---|---|---|
| | phase organique | | | | phase aqueuse | | |
| | entrée | sortie | gain | | entrée | sortie | gain |
| flux n° | 3 | 5 | 5 - 3 | | 10 | 12 | 12 - 10 |
| | kg/h | kg/h | kg/h | | kg/h | kg/h | kg/h |
| MEC | 315 | 370 | | | 0.3 | 18 | |
| MEC-azine | 5416 | 5565 | 150 | | 75 | 38 | -37 |
| MEC-hydrazone | 0 | 0 | | | 89 | 2 | -88 |
| pyrazolines | 5 | 37 | 32 | | 28 | 0 | -28 |
| Acétamide | 46 | 0 | | | 0 | 49 | |
| EAU | 49 | 318 | | | 1137 | 777 | |
| flux total | 7006 | 7401 | | | 1477 | 892 | |

On voit que lors du lavage du flux d'azine 3 par la purge d'hétérocycles de la famille de la pyrazoline 10, les hétérocycles de la famille de la pyrazoline contenus dans la purge 10, sont transférés de façon quasiment quantitative dans le flux d'azine 5 en sortie de la colonne de lavage.

On voit que 150kg/h d'azine sont ainsi récupérés en phase organique (flux 5) lors du lavage du flux 3 par la purge des hétérocycles de la famille de la pyrazoline 10. Aux incertitudes d'analyses près, ceci correspond à la récupération de 37 et 88 kg d'azine et d'hydrazone contenus dans cette purge 10.

Les pyrazolines sont éliminées lors de la purification de l'azine par distillation du flux 5 donnant l'azine distillée 6, en pied de la colonne de distillation avec les résidus lourds.

La solution d'hydrate d'hydrazine (titrant 22.2% en hydrate d'hydrazine ou 14.1% en hydrazine N2H4) obtenue en pied de colonne (flux 11) ne contient plus qu'une très faible concentration en pyrazoline qui n'entraine pas de problèmes de coloration dans la solution d'hydrate d'hydrazine finale obtenue après concentration dans le procédé.

Le bilan est également effectué autour de la colonne d'hydrolyse E et est reporté dans le tableau 2 :

| | | colonne d'hyxdrolyse E | | | | |
|---|---|---|---|---|---|---|
| | azine lavée | azine lavée distillée | purge pyrazoline | MEC recyclée | EAU | Hydrate d'hydrazine |
| flux n° | 5 | 6 | 10 | 8 | 7 | 11 |
| | kg/h | kg/h | kg/h | kg/h | kg/h | kg/h |
| MEC | 370 | 21 | 0.3 | 5206 | | 0 |
| MEC-azine | 5565 | 5523 | 75 | 404 | | 0 |
| MEC-hydrazone | 0 | 0 | 89 | 0 | | 6 |
| pyrazolines | 37 | 3 | 28 | 0 | 0 | 2 |
| Acétamide | 0 | 0 | 0 | 0 | | 0 |
| EAU | 318 | 21 | 1137 | 849 | 11000 | 7768 |
| N2H4 hydrazine | 0 | 0 | 0 | 0 | 0 | 1305 |
| impuretés div QSP | 792 | 136 | 0 | 330 | 0 | 104 |
| flux total | 7401 | 5987 | 1477 | 6789 | 11000 | 9186 |

## Revendications

1. Procédé de préparation d'hydrate d'hydrazine comprenant les étapes suivantes :
(a) on fait réagir l'ammoniac, le peroxyde d'hydrogène et la méthyl éthyl cétone en présence d'une solution comprenant au moins un activateur pour former une azine ;
(b) on sépare à partir du mélange réactionnel issu de l'étape (a) :
- la phase aqueuse comprenant le(s) activateur(s) ; et
- la phase organique comprenant l'azine formée et éventuellement la méthyl éthyl cétone n'ayant pas réagi ;
(c) éventuellement on recycle à l'étape (a) la phase aqueuse après un traitement éventuel ;
(d) on lave, de préférence à contre-courant, la phase organique ;
(e) on distille la phase organique lavée de façon à récupérer l'azine ;
(f) on hydrolyse l'azine pour obtenir de l'hydrate d'hydrazine et régénérer la méthyl éthyl cétone, tout en effectuant une purge des hétérocycles de la famille de la pyrazoline ;
(g) éventuellement on recycle à l'étape (a) la méthyl éthyl cétone obtenue à l'étape (f) ;
(h) on recycle la purge des hétérocycles de la famille de la pyrazoline obtenue à l'étape (f) à l'une au moins des étapes (a), (b), (c), (d) ou (g).

2. Procédé selon la revendication 1, dans lequel on recycle la purge des hétérocycles de la famille de la pyrazoline obtenue à l'étape (f) à l'une au moins des étapes (c), (d) ou (g).

3. Procédé selon la revendication 1 ou 2, dans lequel on recycle la purge des hétérocycles de la famille de la pyrazoline obtenue à l'étape (f) à l'étape (d) de lavage de la phase organique.

4. Procédé selon la revendication 3, dans lequel la purge des hétérocycles de la famille de la pyrazoline est suffisante pour effectuer ledit lavage de la phase organique, sans ajout d'eau.

5. Procédé selon l'une quelconque des revendications précédentes, dans lequel la purge des hétérocycles de la famille de la pyrazoline est réalisée par soutirage, de préférence par un soutirage latéral en continu.

6. Procédé selon l'une quelconque des revendications précédentes, dans lequel l'étape (h) de recyclage est réalisée en continu.

7. Procédé selon l'une quelconque des revendications précédentes, dans lequel les hétérocycles de la famille de la pyrazoline sont la 3,5-diéthyl-5-méthyl-pyrazoline et la 3,4,5-triméthyl-5-éthyl-pyrazoline.

8. Procédé selon l'une quelconque des revendications précédentes, dans lequel l'étape (f) d'hydrolyse de l'azine et de régénération de la méthyl éthyl cétone est effectuée dans une colonne de distillation à garnissage ou à plateaux, de préférence fonctionnant sous une pression de 2 à 25 bars et avec une température de pied de 150 °C à 200 °C.

9. Procédé selon la revendication 8, dans lequel la teneur en hétérocycles de la famille de la pyrazoline est inférieure ou égale à 5 % en poids, plus préférentiellement comprise entre 1% et 3% en poids, par rapport au poids total de la phase liquide présente sur les plateaux de la colonne ou dans les parties de la colonne à hydrolyse où ladite teneur est à son maximum.

10. Procédé selon l'une quelconque des revendications précédentes, dans lequel ledit activateur est l'acétamide.

## Patentansprüche

1. Verfahren zur Herstellung von Hydrazinhydrat, das die folgenden Schritte umfasst:
(a) das Umsetzen von Ammoniak, Wasserstoffperoxid und Methylethylketon in Gegenwart einer mindestens einen Aktivator umfassenden Lösung, wodurch ein Azin gebildet wird;
(b) das Abtrennen von der Reaktionsmischung aus Schritt (a):
- der den (die) Aktivator (en) enthaltenden wässrigen Phase und
- der organischen Phase, die das gebildete Azin und gegebenenfalls nicht umgesetztes Methylethylketon umfasst;
(c) gegebenenfalls das Zurückführen der wässrigen Phase nach einer etwaigen Behandlung in Schritt (a);
(d) das Waschen, vorzugsweise im Gegenstrom, der organischen Phase;
(e) das Destillieren der gewaschenen organischen Phase so, dass das Azin zurückgewonnen wird;
(f) das Hydrolysieren des Azins, wodurch Hydrazinhydrat erhalten wird, und das Regenerieren von Methylethylketon, wobei Heterocyclen der Pyrazolin-Familie abgeführt werden;
(g) gegebenenfalls das Zurückführen des in Schritt (f) erhaltenen Methylethylketons in Schritt (a);
(h) das Zurückführen der in Schritt (f) erhaltenen Abführung von Heterocyclen der Pyrazolin-Familie in mindestens einen der Schritte (a), (b), (c), (d) oder (g).

2. Verfahren nach Anspruch 1, wobei die in Schritt (f) erhaltene Abführung von Heterocyclen der Pyrazolin-Familie in mindestens einen der Schritte (c), (d) oder (g) zurückgeführt werden.

3. Verfahren nach Anspruch 1 oder 2, wobei die in Schritt (f) erhaltene Abführung von Heterocyclen der Pyrazolin-Familie in Schritt (d) des Waschens der organischen Phase zurückgeführt werden.

4. Verfahren nach Anspruch 3, wobei die Abführung von Heterocyclen der Pyrazolin-Familie ausreichend ist, um das Waschen der organischen Phase zu bewirken, ohne dass Wasser zugegeben wird.

5. Verfahren nach einem der vorhergehenden Ansprüche, wobei die Abführung von Heterocyclen der Pyrazolin-Familie durch Abziehen, vorzugsweise mittels eines kontinuierlichen Seitenabzugs, erfolgt.

6. Verfahren nach einem der vorhergehenden Ansprüche, wobei der Rückführungsschritt (h) kontinuierlich erfolgt.

7. Verfahren nach einem der vorhergehenden Ansprüche, wobei es sich bei den Heterocyclen der Pyrazolin-Familie um 3,5-Diethyl-5-methylpyrazolin und 3,4,5-Trimethyl-5-ethylpyrazolin handelt.

8. Verfahren nach einem der vorhergehenden Ansprüche, wobei der Schritt (f) der Hydrolyse des Azins und der Regeneration des Methylethylketons in einer Füllkörper- oder Boden-Destillationskolonne erfolgt, die vorzugsweise unter einem Druck von 2 bis 25 bar und mit einer Sumpftemperatur von 150 °C bis 200 °C betrieben wird.

9. Verfahren nach Anspruch 8, wobei der Gehalt an Heterocyclen der Pyrazolin-Familie kleiner oder gleich 5 Gew.-% ist und weiter bevorzugt zwischen 1 und 3 Gew.-% liegt, bezogen auf das Gesamtgewicht der flüssigen Phase, die auf den Böden der Kolonne oder in den Teilen der Hydrolysekolonne vorhanden ist, wo der Gehalt am höchsten ist.

10. Verfahren nach einem der vorhergehenden Ansprüche, wobei es sich bei dem Aktivator um Acetamid handelt.

## Claims

1. Process for preparing hydrazine hydrate, comprising the following steps:
(a) reacting ammonia, hydrogen peroxide and methyl ethyl ketone in the presence of a solution comprising at least one activator to form an azine;
(b) treating the reaction mixture from step (a) to isolate:
- the aqueous phase comprising the activator(s); and
- the organic phase comprising the resulting azine and optionally the unreacted methyl ethyl ketone;
(c) optionally recycling the aqueous phase to step (a) after optional treatment;
(d) washing the organic phase, preferably in countercurrent;
(e) distilling the washed organic phase to recover the azine;
(f) hydrolyzing the azine to give hydrazine hydrate and regenerate methyl ethyl ketone, with pyrazoline-class heterocycles being purged;
(g) optionally recycling the methyl ethyl ketone obtained in step (f) to step (a);
(h) recycling the pyrazoline-class heterocycles purge obtained in step (f) to one at least of steps (a), (b), (c), (d) or (g).

2. Process according to Claim 1, wherein the pyrazoline-class heterocycles purge obtained in step (f) is recycled to one at least of steps (c), (d) or (g).

3. Process according to Claim 1 or 2, wherein the pyrazoline-class heterocycles purge obtained in step (f) is recycled to the organic phase washing step (d).

4. Process according to Claim 3, wherein the pyrazoline-class heterocycles purge is sufficient to perform said organic phase washing, without addition of water.

5. Process according to any one of the preceding claims, wherein the pyrazoline-class heterocycles purge is carried out by withdrawal, preferably by a continuous side takeoff.

6. Process according to any one of the preceding claims, wherein the recycling step (h) is carried out continuously.

7. Process according to any one of the preceding claims, wherein the pyrazoline-class heterocycles are 3,5-diethyl-5-methylpyrazoline and 3,4,5-trimethyl-5-ethylpyrazoline.

8. Process according to any one of the preceding claims, wherein the azine hydrolysis and methyl ethyl ketone regeneration step (f) is performed in a packed or tray distillation column, preferably operating at a pressure of 2 to 25 bar and with a bottom temperature of 150°C to 200°C.

9. Process according to Claim 8, wherein the amount of pyrazoline-class heterocycles is not more than 5% by weight, more preferably between 1% and 3% by weight, relative to the total weight of the liquid phase on the trays of the column or in the parts of the hydrolysis column at which said amount is at its maximum.

10. Process according to any one of the preceding claims, wherein said activator is acetamide.
